# DEMANDE DE BREVET EUROPEEN

(11) **EP 4 371 499 A1**
(43) Date de publication de la demande: **22.05.2024**
(21) Numéro de dépôt: 23209399.7
(22) Date de dépôt: 13.11.2023
(51) Int. Cl.: A61B 6/10, G01B 7/02

(54) **DISPOSITIF MULTI ÉLECTRODES DE DÉTECTION D'OBJETS**

(30) Priorité: 15.11.2022 FR 2211874
(71) Demandeur: FOGALE SENSORS, 30900 Nîmes (FR)
(72) Inventeur: CHAKOUR, Yacine, 30210 SERNHAC (FR); ROZIERE, Didier, 30900 NÎMES (FR); NEEL, Christian, 30900 NÎMES (FR)
(74) Mandataire: IPAZ

(57) **Abrégé**

Dispositif de détection d'objets comprenant un capteur de proximité, ledit capteur de proximité comprend deux électrodes de mesure, chacune des électrodes de mesure du capteur de proximité sont comprises sur une même face du capteur de proximité, les deux électrodes de mesure sont décalées le long d'une direction latérale. Le dispositif de détection d'objets comprend, en outre, une unité de traitement agencée pour détecter, par analyse des signaux provenant des deux électrodes de mesures décalées le long de la direction latérale, la présence d'un objet situé latéralement par rapport au capteur de proximité.

## Description

### Domaine technique

La présente invention appartient au domaine technique des systèmes anticollisions, de positionnement et de contournement, en particulier pour machines médicales.

L'invention concerne les dispositifs de mesure et de contrôle de la position, dans plusieurs directions, de machines médicales appartenant, en particulier, au domaine de l'imagerie et du traitement médical et notamment les machines médicales utilisant un bras en C ou « C-Arm ».

L'invention porte notamment sur les dispositifs de mesure et de contrôle de position comprenant un capteur capacitif multi-électrodes.

### Etat de la technique antérieure

Les systèmes anticollisions et de positionnement de l'état de la technique comprennent plusieurs capteurs ou détecteurs de proximité comprenant chacun un ensemble d'électrodes capacitives. Tous les systèmes anticollisions ont pour but d'améliorer la sécurité du sujet à imager en évitant une collision entre la machine médicale ou le bras en C et le sujet à imager. En outre, tous les systèmes anticollisions et de positionnement ont également pour but d'améliorer le positionnement ou le contournement de la machine médicale ou du bras en C par rapport au sujet à imager et à l'environnement.

Les systèmes anticollisions et de positionnement de l'état de la technique sont équipés de plusieurs capteurs de proximité. Les capteurs de proximité comprennent une surface de détection en vis-à-vis de laquelle la présence d'un objet sera détectée. Les systèmes d'anticollision et de positionnement de l'état de la technique comprennent des capteurs de proximité recouvrant chacun une surface d'au moins une partie de la machine médicale qui est perpendiculaire à un sens de déplacement de la machine médicale. Ainsi, pour chaque sens de déplacement de la partie de la machine médicale, la surface de détection d'un des capteurs de proximité fait face à un sens de déplacement selon laquelle la partie de la machine est susceptible de se déplacer pour détecter les objets se rapprochant de la partie de la machine médicale selon ce sens de déplacement. Un tel système est décrit, par exemple, dans le document de l'état de l'art WO2004/023067.

Aussi, pour un bras en C agencé pour se déplacer selon chacun des sens de trois axes, autrement dit selon chaque sens de trois directions, le système anticollision comporte, a minima, cinq capteurs de proximité ; le déplacement selon la direction reliant le bras en C au patient dans le sens s'étendant depuis le patient vers le bras en C ne nécessitant pas de détection.

Les améliorations et les efforts de développement apportés aux systèmes anticollisions et de positionnement de l'état de la technique portent sur l'optimisation de la position des détecteurs par rapport au patient. Cela a pour but d'améliorer la qualité d'image, de réduire les doses de rayonnements et de procurer une meilleure ergonomie.

Un but de l'invention est, en outre, de proposer un dispositif de détection d'objet et un procédé de détection associé permettant :
- d'améliorer :
   - la sécurité du sujet à imager et des objets environnant la machine médicale, et/ou
   - le positionnement de la machine médicale ou du bras en C par rapport au sujet à imager et à l'environnement,
   - le contournement du sujet à imager par la machine médicale ou le bras en C, et/ou
- de diminuer l'encombrement du dispositif de détection d'objet, et/ou
- de diminuer le coût du dispositif de détection d'objet, et/ou
- de faciliter la fabrication du dispositif de détection d'objet, et/ou
- de faciliter l'intégration du dispositif de détection d'objet sur la machine médicale.

### Présentation de l'invention

A cet effet, il est proposé un dispositif de détection d'objets comprenant un capteur de proximité, ledit capteur de proximité comprend deux électrodes de mesure ou plus, chacune des électrodes de mesure sont comprises ou disposées ou situées sur une même face ou sur un même côté du capteur de proximité, deux électrodes de mesure sont décalées le long d'au moins une direction, de préférence le long d'au moins une direction latérale, de préférence encore le long d'une direction latérale, dite première direction latérale, selon laquelle les deux électrodes de mesure sont alignées.

Le dispositif de détection d'objets comprend, en outre, une unité de traitement agencée et/ou configurée et/ou programmée pour détecter, par analyse des signaux provenant des deux électrodes de mesures décalées le long de l'au moins une direction, de préférence le long de la première direction latérale, la présence d'un objet, situé selon une direction, dite verticale, orthogonale à la face du capteur de proximité comprenant les électrodes de mesure et/ou latéralement, de préférence selon l'au moins une direction latérale, de préférence encore selon toute direction latérale ou selon une direction latérale quelconque, de manière avantageuse, et en particulier, selon la première direction latérale, par rapport au capteur de proximité et/ou au dispositif de détection d'objets.

De préférence, il est entendu par électrode de mesure une électrode capacitive ou électrode capacitive de mesure.

De préférence, il est entendu par signal provenant d'une électrode de mesure un signal mesuré qui est représentatif d'une capacité, dite électrode-objet. De préférence, ce signal de mesure correspond à la capacité électrode-objet détectée ou mesurée par l'électrode de mesure.

De préférence, il est entendu par direction latérale ou latéralement toute direction qui est principalement parallèle à une direction selon laquelle s'étend principalement la face ou le côté du capteur de proximité sur lequel sont comprises chacune des électrodes de mesure.

De préférence, les électrodes de mesure du capteur de proximité sont comprises ou situées ou disposées uniquement sur une même face ou sur un même côté du dispositif de détection. De préférence, la face ou le côté sur lequel sont comprises les électrodes de mesure est destiné à être orienté ou disposé principalement perpendiculairement à la direction latérale.

De préférence, une face de détection ou face sensible de chaque électrode de mesure du capteur de proximité est orientée principalement selon une même direction, de préférence selon la direction verticale.

La direction orthogonale à la face du capteur de proximité comprenant les électrodes de mesure est, de préférence, verticale et, dans ce cas, la direction latérale par rapport au capteur de proximité est horizontale. Toutefois, la direction orthogonale à la face du capteur de proximité comprenant les électrodes de mesure peut être quelconque. Par exemple, la direction orthogonale à la face du capteur de proximité comprenant les électrodes de mesure peut ne pas être verticale mais être horizontale et, dans ce cas, la direction latérale par rapport au capteur de proximité est verticale.

De préférence, il est entendu par verticalement une direction verticale quelconque.

Aussi, même lorsque le capteur est immobile, et quelle que soit la direction selon laquelle un objet entre dans la zone de détection du capteur de proximité, il est possible de déterminer, a minima approximativement, ou d'obtenir une information, a minima approximative, de la zone d'espace dans laquelle se trouve l'objet détecté sans qu'il soit nécessaire de disposer de capteurs sur une face latérale ou une extrémité latérale ou un côté latéral ou un bord latéral du capteur de proximité.

De préférence, le capteur de proximité est compris ou s'étend, de préférence encore les électrodes de mesure sont comprises ou s'étendent, dans un plan ou dans une surface convexe ou concave.

Il peut être entendu par un objet situé verticalement par rapport au dispositif de détection d'objets et/ou au capteur de proximité un objet situé du côté, mais pas en regard ou en vis-à-vis, de la face de détection ou face sensible d'une électrode de mesure et/ou de la face du capteur de proximité sur lequel sont comprises chacune des électrodes de mesure. Il peut être entendu par un objet situé verticalement par rapport au dispositif de détection d'objets et/ou au capteur de proximité un objet situé en regard ou en vis-à-vis de la face de détection ou face sensible d'une électrode de mesure et/ou de la face ou du côté du capteur de proximité sur lequel sont comprises chacune des électrodes de mesure. Il peut être entendu par un objet situé latéralement au dispositif de détection d'objets et/ou au capteur de proximité un objet situé d'un côté, mais pas en regard ou en vis-à-vis, d'une face latérale ou d'une extrémité latérale ou d'un côté latéral ou d'un bord latéral du dispositif de détection d'objets et/ou d'une face latérale ou d'une extrémité latérale ou d'un côté latéral ou d'un bord latéral du capteur de proximité. Il peut être entendu par un objet situé latéralement au dispositif de détection d'objets un objet situé en regard ou en vis à vis d'une face latérale ou d'une extrémité latérale ou d'un côté latéral ou d'un bord latéral du dispositif de détection d'objets et/ou d'une face latérale ou d'une extrémité latérale ou d'un côté latéral ou d'un bord latéral du capteur de proximité.

De préférence, l'unité de traitement est agencée pour détecter, par analyse des signaux provenant des deux électrodes de mesures décalées le long de la direction latérale, une direction, de préférence une direction latérale, de déplacement relatif d'un objet.

Il peut être entendu par déplacement relatif d'un objet un déplacement d'un objet relativement au capteur de proximité.

De préférence, l'unité de traitement est agencée pour détecter, par analyse des signaux provenant des deux électrodes de mesures décalées le long de la direction latérale, un déplacement relatif d'un objet selon la direction latérale selon laquelle sont décalées les deux électrodes.

De préférence, et sans que cela signifie que le dispositif selon l'invention ne soit pas apte à l'atteindre, le but recherché n'est pas uniquement une détection de la position de l'objet. De préférence, le but recherché est une détection de la direction de déplacement relatif de l'objet.

De préférence, le capteur de proximité comprend au moins trois électrodes de mesure parmi lesquelles trois électrodes de mesure sont agencées ou disposées de sorte à former un triangle.

De préférence, l'unité de traitement est agencée pour détecter, par analyse des signaux provenant d'au moins deux électrodes de mesure parmi les trois électrodes de mesure, du capteur de proximité, formant un triangle, de préférence par analyse des signaux provenant des deux électrodes décalées selon la première direction latérale, de préférence encore par analyse des signaux provenant des trois électrodes de mesure formant un triangle, de manière avantageuse, par analyse des signaux provenant de chacune des au moins trois électrodes de mesure, la présence d'un objet situé verticalement et/ou latéralement par rapport au dispositif de détection et/ou au capteur de proximité.

De préférence, l'agencement en triangle de trois électrodes de mesure du capteur de proximité permet de former trois couples d'électrodes de mesure décalées chacun le long d'une direction latérale, selon laquelle les deux électrodes de mesure d'un couple de mesure sont alignées, différente. De préférence, chaque électrode de mesure appartient à deux couples d'électrodes de mesure ou plus.

De préférence, l'agencement des trois électrodes de mesure formant un triangle permet qu'au moins une électrode de mesure ne soit pas alignée avec les deux électrodes de mesure, de préférence encore avec les deux électrodes de mesures décalées le long de l'au moins une direction, de préférence le long de la première direction latérale.

De préférence, l'unité de traitement est agencée, en outre, pour détecter, par analyse des signaux provenant d'au moins deux électrodes de mesure ou de chacune des électrodes de mesure du capteur de proximité, la présence d'un objet situé verticalement et/ou latéralement par rapport au capteur de proximité et/ou au dispositif de détection d'objets.

De préférence, les trois électrodes de mesure formant un triangle sont disposées de sorte à former un triangle rectangle, de préférence un triangle équilatéral.

De préférence, au moins deux électrodes de mesure ne sont pas alignées avec au moins deux autres électrodes de mesure. De préférence, les deux électrodes de mesure alignées selon la première direction latérale ne sont pas alignées avec deux autres électrodes de mesure décalées le long d'une direction latérale, dite deuxième direction latérale. De préférence, la première et la deuxième direction forme un angle non nul entre elles. De préférence, la première et la deuxième direction latérale sont orthogonales.

De préférence, le capteur de proximité comprend quatre électrodes de mesure ou plus parmi lesquelles deux électrodes de mesure sont décalées le long de la deuxième direction latérale. De préférence, l'unité de traitement est agencée, en outre, pour détecter, par analyse des signaux provenant des quatre électrodes de mesure, la présence d'un objet situé verticalement et/ou latéralement par rapport au dispositif de détection d'objets et/ou au capteur de proximité.

De préférence, l'unité de traitement est agencée pour détecter, par analyse des signaux provenant d'au moins deux électrodes de mesure du dispositif de détection, de préférence encore des au moins trois électrodes de mesures, de manière avantageuse à partir des trois électrodes de mesure disposées de sorte à former un triangle, une direction, de préférence une direction latérale, de déplacement relatif d'un objet.

De préférence, au moins une électrode de mesure parmi les trois électrodes de mesure formant un triangle détecte un objet situé latéralement, selon une direction latérale quelconque, préalablement à au moins une autre des électrodes de mesure formant un triangle. Ainsi, de préférence, un tel agencement des électrodes de mesure permet que le signal provenant d'au moins deux électrodes de mesure soit différents pour tout objet situé latéralement au capteur de mesure. Pour tout objet situé verticalement au capteur de mesure, au moins une électrode parmi les trois électrodes de mesure formant un triangle détecte un objet situé verticalement, selon une direction verticale quelconque, préalablement à au moins une autre des électrodes de mesure formant un triangle à l'exception d'un objet situé verticalement le long de l'axe de traitement, qui est situé à équidistance des trois électrodes, dans ce cas les trois électrodes de mesure formant un triangle détectent simultanément l'objet. Ainsi, pour tout objet situé verticalement au capteur de mesure, un tel agencement des électrodes de mesure permet qu'au moins un des signaux de mesure soit différent d'au moins un autre des signaux de mesure à l'exception d'un objet situé verticalement le long de l'axe de traitement, dans ce cas les signaux de mesure provenant des trois électrodes de mesure formant un triangle sont identiques.

Aussi, lorsqu'il est immobile, un capteur de proximité comprenant trois électrodes de mesure formant un triangle permet d'améliorer la détermination approximative de la zone d'espace dans laquelle se trouve l'objet détecté comparé à un capteur de proximité comprenant deux électrodes de mesure décalées le long d'une direction latérale.

De préférence, le capteur de proximité et/ou le dispositif de détection d'objet est déplaçable verticalement et/ou est déplaçable latéralement, de préférence selon au moins une direction latérale, de préférence encore selon toute direction latérale. De préférence, le capteur de proximité et/ou le dispositif de détection d'objet est déplaçable selon toute direction, par exemple selon une direction comprenant une combinaison de composante(s) verticale(s) et latérale(s).

Il peut être entendu par déplacement relatif d'un objet un déplacement d'un objet relativement au capteur de proximité et/ou un déplacement du capteur de proximité relativement à un objet.

De préférence, le capteur de proximité est destiné à être monté sur, ou à être solidaire de, une tête fonctionnelle. De préférence, le capteur de proximité est destiné à être disposé en regard d'une face, dite face d'émission, de la tête fonctionnelle. De préférence, la tête fonctionnelle et/ou le capteur de proximité est destinée à être positionnée en direction d'un objet, par exemple un objet à imager, à analyser, à traiter ou à irradier. De préférence, la tête fonctionnelle est déplaçable verticalement et/ou est déplaçable latéralement, de préférence selon au moins une direction latérale, de préférence encore selon toute direction latérale.

L'objet détecté par le dispositif de détection d'objets peut être l'objet à imager et/ou tout autre objet présent dans la zone de détection du détecteur de proximité.

De préférence, la tête fonctionnelle et/ou le capteur de proximité est déplaçable verticalement et/ou latéralement par translation et/ou par rotation.

De préférence, le capteur de proximité et/ou le dispositif de détection d'objets est destiné à être mis en mouvement ou déplacé verticalement et/ou latéralement par la tête fonctionnelle. Ainsi, toute caractéristique se rapportant au déplacement de la tête fonctionnelle est transposable au dispositif de détection d'objet, au capteur de proximité et aux électrodes de mesure et inversement.

De préférence, le capteur de proximité comprend une ouverture centrale.

De préférence, au moins deux électrodes de mesure sont disposées de part et d'autre de l'ouverture centrale. De préférence, les électrodes de mesure sont disposées, en couple, de part et d'autre de l'ouverture centrale.

De préférence, le capteur de proximité est agencé pour qu'une, plusieurs ou l'ensemble des électrodes de mesure s'étendent, de préférence en partie, radialement, par rapport à un centre du capteur de proximité, de préférence par rapport à l'ouverture centrale, au-delà de la face d'émission de la tête fonctionnelle.

De préférence, une, plusieurs ou l'ensemble des électrodes de mesure sont disposées en périphérie ou sur un pourtour du capteur de proximité.

De préférence, un bord ou un côté d'une, de plusieurs ou de chaque des électrodes de mesure peut former ou constituer une face latérale ou une extrémité latérale ou un côté latéral ou un bord latéral du capteur de proximité.

De préférence, le dispositif de détection d'objets et/ou le capteur de proximité est agencé pour qu'une, plusieurs ou l'ensemble des électrodes de mesure soient disposées ou situées en périphérie ou sur un pourtour de la face d'émission de la tête fonctionnelle.

De préférence, la face de détection d'une électrode de mesure, de préférence de chacune des électrodes de mesure, est destinée à être orientée dans le sens reliant la face d'émission de la tête fonctionnelle au capteur de proximité ou à l'objet à imager. De préférence, la face de détection d'une électrode de mesure, de préférence de chacune des électrodes de mesure, est destinée à être orientée en direction de l'objet à imager.

De préférence, l'unité de traitement est agencée pour détecter la présence d'un objet par analyse :
- de signaux individuels d'au moins deux électrodes de mesure ou, de préférence, de chacune des électrodes de mesure, du capteur de proximité, et/ou
- de signaux groupés obtenus en sommant des signaux provenant d'au moins deux électrodes de mesure ou, de préférence, de chacune des électrodes de mesure du capteur de proximité, et/ou
- de signaux différenciés obtenus en soustrayant des signaux provenant d'au moins deux électrodes de mesure ou, de préférence, de chacune des électrodes de mesure du capteur de proximité, et/ou
- de signaux individuels et/ou de signaux groupés et/ou de signaux différenciés.

De préférence, l'unité de traitement est agencée pour, à partir des signaux provenant des électrodes de mesure du capteur de proximité, de préférence à partir d'au moins deux signaux provenant d'au moins deux électrodes de mesure distinctes, par exemple les deux électrodes de mesure décalées selon la première direction latérale, de préférence encore à partir d'au moins trois signaux provenant d'au moins deux électrodes de mesure parmi les au moins trois électrodes ou d'au moins deux électrodes de mesure parmi les trois électrodes de mesure formant un triangle, de manière avantageuse d'au moins trois électrodes de mesure distinctes, de manière préférée à partir d'au moins trois électrodes de mesure, par exemple des trois électrode formant un triangle, de manière encore préférée à partir d'au moins quatre signaux provenant d'au moins autre électrodes de mesure distinctes, par exemple les deux électrodes de mesure décalées selon la première direction latérale et les deux électrodes de mesure décalées selon la deuxième direction latérale, de manière particulièrement préférée à partir de chacun des signaux provenant de chacune des électrodes de mesure, et de données relatives à un déplacement du capteur de proximité, déterminer la position d'un objet et/ou le déplacement d'un objet relativement au capteur de proximité.

Selon l'invention, il peut être entendu par signaux provenant d'au moins deux électrodes de mesure distinctes des signaux provenant :
- des deux électrodes de mesure décalées selon la première direction latérale, et/ou
- des deux électrodes de mesure décalées selon la deuxième direction latérale, et/ou
- de deux électrodes de mesure parmi les au moins trois électrodes de mesure, et/ou
- de deux électrodes de mesure parmi les trois électrodes de mesure formant un triangle, et/ou
- d'au moins trois électrodes de mesure, et/ou
- des trois électrodes de mesure formant un triangle, et/ou
- d'au moins quatre électrodes de mesure, et/ou
- de quatre électrodes de mesure, par exemple des deux électrodes de mesure décalées selon la première direction latérale et des deux électrodes de mesure décalées selon la deuxième direction latérale, et/ou
- de chacune des électrodes de mesure du capteur de proximité.

La détermination de la position d'un objet relativement au capteur de proximité et/ou d'un mouvement d'un objet relativement au capteur de proximité est améliorer lorsque des données relatives à un mouvement ou un déplacement du capteur de proximité et/ou du dispositif de détection et/ou de la tête fonctionnelle sont disponibles. A la différence du cas où le capteur de proximité est immobile et qu'une détermination de la zone d'espace dans laquelle se trouve l'objet détecté est réalisée, le déplacement du capteur de proximité permet la détermination de la position d'un objet relativement au capteur de proximité et/ou d'un mouvement d'un objet relativement au capteur de proximité.

Le dispositif de détection d'objets peut également être destiné à être monté sur, ou à être solidaire de, un système, par exemple un système d'imagerie ou un système d'analyse ou un système de traitement médical, par exemple pour la radiothérapie, ou un système de mise en mouvement, ou d'un dispositif, tel qu'un robot ou un bras articulé, par exemple six axes.

Selon l'invention, il est également proposé une utilisation du dispositif de détection d'objets selon l'invention pour le contrôle et/ou la commande et/ou le positionnement, par exemple au moyen de l'unité de traitement du dispositif de détection d'objets, d'un système, par exemple un système d'imagerie ou un système d'analyse ou un système de traitement médical, par exemple pour la radiothérapie, ou un système de mise en mouvement, ou d'un dispositif, tel qu'un robot ou un bras articulé, par exemple six axes.

Selon l'invention, il est également proposé un système comprenant :
- une tête fonctionnelle comprenant une face d'émission destinée à être positionnée en direction d'un objet à imager ou à analyser,
- un capteur de proximité disposé ou monté ou solidaire en regard de la face d'émission de la tête fonctionnelle, ledit capteur de proximité comprend au moins deux électrodes de mesure parmi lesquelles deux électrodes de mesure sont décalées le long d'une direction latérale, chacune des électrodes de mesure du capteur de proximité sont comprises sur une même face du capteur de proximité.

La tête fonctionnelle est déplaçable selon une direction, dite verticale, orthogonale à la face du capteur comprenant les électrodes de mesure et déplaçable latéralement. Le système comprend une unité de traitement agencée pour détecter, par analyse des signaux provenant des deux électrodes de mesure décalées le long de la direction latérale, la présence d'un objet situé latéralement par rapport au détecteur de proximité.

De préférence, le système selon l'invention peut être un système d'imagerie, par exemple médical, ou un système d'analyse, par exemple d'analyse médicale, ou un système de traitement médical, par exemple pour la radiothérapie, ou un système de mise en mouvement ou un dispositif, par exemple un robot ou un bras articulé, de préférence six axes.

Le dispositif de détection du système peut comprendre un unique capteur de proximité.

Le dispositif de détection du système peut comprendre plusieurs capteurs de proximité.

De préférence, l'unité de traitement est agencée pour détecter, par analyse des signaux provenant des au moins deux électrodes de mesures, une direction de déplacement relatif d'un objet.

De préférence, l'unité de traitement est agencée pour, à partir des signaux provenant des électrodes de mesure du capteur de proximité, de préférence des signaux provenant d'au moins deux électrodes de mesure, et de données relatives à un déplacement de la tête fonctionnelle, provenant par exemple de l'unité de traitement, déterminer la position d'un objet et/ou le déplacement d'un objet relativement au capteur de proximité.

De préférence, le système comprend une unité de commande agencée pour, à partir des signaux provenant des électrodes de mesure du capteur de proximité, de préférence des signaux provenant d'au moins deux électrodes de mesure, et/ou à partir de données provenant de l'unité de traitement, par exemple à partir de données, provenant par exemple de l'unité de commande, de position ou de déplacement de la tête fonctionnelle et/ou du capteur de proximité, positionner le capteur de proximité et/ou le la tête fonctionnelle, de préférence à une distance donnée de l'objet à imager et/ou maintenir une distance minimale entre le capteur de proximité et/ou la tête fonctionnelle et l'objet à imager et/ou immobiliser la tête fonctionnelle, par exemple en cas de risque de collision ou de la présence d'un objet à proximité immédiate.

De préférence, l'unité de commande est agencée pour contrôler et/ou commander la tête fonctionnelle.

De préférence, l'unité de commande est agencée pour empêcher ou prévenir la collision de la tête fonctionnelle et/ou du capteur de proximité avec un objet environnant, c'est à dire présent dans la zone de détection du capteur de proximité, ou avec l'objet à imager.

De préférence, l'unité de commande est agencée pour contrôler la tête fonctionnelle, de préférence les déplacements de la tête fonctionnelle.

De préférence, l'unité de commande est agencée pour commander un moyen de mise en mouvement de la tête fonctionnelle. De préférence, le moyen de moyen de mise en mouvement de la tête fonctionnelle est agencé pour positionner et/ou maintenir et/ou immobiliser la tête fonctionnelle.

De préférence, le capteur de proximité du système selon l'invention est le capteur de proximité du dispositif de détection d'objets selon l'invention. De préférence, l'unité de traitement du système selon l'invention est l'unité de traitement du dispositif de détection d'objets selon l'invention. De préférence, le système comprend le dispositif de détection d'objets selon l'invention. Toute caractéristique du dispositif de détection d'objets, entre autres les caractéristiques des électrodes de mesure et du détecteur de proximité, selon l'invention est directement transposable au système selon l'invention et inversement.

Selon l'invention, il est également proposé un procédé de détection d'objets mise en oeuvre par un dispositif de détection d'objets comprenant un capteur de proximité, ledit capteur de proximité comprend deux électrodes de mesure, chacune des électrodes de mesure du capteur de proximité sont comprises sur une même face du capteur de proximité, les deux électrodes de mesure sont décalées le long d'une direction latérale ; ledit procédé comprend les étapes consistant à :
- mesurer les signaux provenant des deux électrodes de mesure du capteur de proximité,
- analyser, par une unité de traitement du dispositif de détection, les deux signaux provenant des deux électrodes de mesure décalées le long de la direction latérale pour détecter la présence d'un objet situé selon une direction, dite verticale, orthogonale à la face du capteur comprenant les électrodes de mesure et/ou latéralement par rapport au détecteur de proximité.

Le procédé comprend l'étape consistant à, ou l'étape de mesure des signaux consiste à, mesurer au moins deux signaux, de préférence au moins trois signaux, provenant chacun d'une électrode de mesure distincte parmi au moins trois électrodes de mesure du capteur de proximité, trois électrodes de mesure du capteur de proximité sont disposées de sorte à former un triangle.

Le procédé comprend l'étape consistant à, ou l'étape d'analyse des signaux consiste à, analyser, par l'unité de traitement du dispositif de détection, les signaux provenant d'au moins deux électrodes de mesure parmi les trois électrodes du capteur de proximité formant un triangle, de préférence les signaux provenant des trois électrodes du capteur de proximité formant un triangle, pour détecter la présence d'un objet situé latéralement par rapport au détecteur de proximité.

De préférence, le procédé comprend l'étape de détermination d'une direction de déplacement relatif d'un objet par analyse, par l'unité de traitement du dispositif de détection (1), d'au moins deux signaux provenant de deux électrodes de mesure (5) distinctes du capteur de proximité (2)

De préférence, la détection de la présence d'un objet situé latéralement par rapport au détecteur de proximité est réalisée par analyse :
- de signaux individuels d'au moins deux électrodes de mesure ou de chacune des électrodes de mesure du capteur de proximité, et/ou
- de signaux groupés obtenus en sommant des signaux provenant d'au moins deux électrodes de mesure ou de chacune des électrodes de mesure du capteur de proximité, et/ou
- de signaux différenciés obtenus en soustrayant des signaux provenant d'au moins deux électrodes de mesure ou de chacune des électrodes de mesure du capteur de proximité, et/ou
- de signaux individuels et/ou de signaux groupés et/ou de signaux différenciés.

De préférence, le procédé comprend l'étape consistant à déterminer la position d'un objet et/ou le déplacement d'un objet relativement au capteur de proximité par analyse des signaux provenant des électrodes de mesure du capteur de proximité.

De préférence, le capteur de proximité et/ou la tête fonctionnelle est déplaçable verticalement et est déplaçable latéralement.

Le procédé comprend l'étape consistant à déterminer la position d'un objet relativement au capteur de proximité par analyse des signaux provenant des électrodes de mesure du capteur de proximité, de préférence des signaux provenant d'au moins deux électrodes de mesure, et de données relatives à un déplacement du capteur de proximité.

De préférence, les données relatives au déplacement sont obtenues par mise en mouvement du capteur de proximité et/ou du dispositif de détection et/ou de la tête fonctionnelle. De préférence, les données relatives au déplacement sont obtenues, ou la mise en mouvement du capteur de proximité et/ou du dispositif de détection et/ou de la tête fonctionnelle est réalisée, lorsqu'un objet est dans la zone de détection du capteur de proximité.

De préférence, le procédé de détection d'objets est mis en oeuvre par le système selon l'invention, et, de préférence, le capteur de proximité est destiné à être monté sur une tête fonctionnelle et à être disposé en regard d'une face, dite face d'émission, de la tête fonctionnelle. La tête fonctionnelle est destinée à être positionnée en direction d'un objet à imager ou à analyser et est déplaçable verticalement et est déplaçable latéralement.

Le procédé comprend l'étape consistant à, à partir des signaux provenant des électrodes de mesure du capteur de proximité, de préférence des signaux provenant d'au moins deux électrodes de mesure, générer des données, par l'unité de traitement, destinées à être utilisées par une unité de commande, pour :
- maintenir une distance minimale entre le capteur de proximité et l'objet à imager, et/ou
- positionner le capteur de proximité à une distance donnée de l'objet à imager, et/ou
- immobiliser la tête fonctionnelle.

De préférence, l'étape consistant à générer des données est mise en oeuvre à partir de signaux provenant des électrodes de mesure du capteur de proximité, de préférence des signaux provenant d'au moins deux électrodes de mesure ou de chacune des électrodes de mesure du capteur de proximité, et/ou à partir de données provenant de l'unité de traitement, par exemple à partir de données, provenant par exemple de l'unité de commande, de position ou de déplacement de la tête fonctionnelle.

De préférence, le procédé comprend l'étape consistant à empêcher ou prévenir, au moyen de l'unité de commande, la collision de la tête fonctionnelle et/ou du capteur de proximité avec un objet environnant ou avec l'objet à imager.

De préférence, le procédé comprend l'étape consistant à contrôler, au moyen de l'unité de commande, la tête fonctionnelle. De préférence, le procédé comprend l'étape consistant à contrôler, au moyen de l'unité de commande, les déplacements de la tête fonctionnelle.

De préférence, le détecteur de proximité, le dispositif de détection d'objets et le système selon l'invention, conviennent, de préférence encore sont particulièrement adaptés, de manière davantage préférée sont conçues et de manière particulièrement avantageuse sont spécialement conçues, pour mettre en oeuvre le procédé selon l'invention.

Toute caractéristique du détecteur de proximité, du dispositif de détection d'objets et du système selon l'invention est directement transposable au procédé de détection d'objets selon l'invention et inversement.

### Description des figures

D'autres avantages et particularités de l'invention apparaîtront à la lecture de la description détaillée de mises en oeuvre et de modes de réalisation nullement limitatifs, et des dessins annexés suivants :
[Fig. 1] la FIGURE 1 est une représentation schématique en vue de coupe d'un mode de réalisation d'un capteur de proximité selon l'invention,
[Fig. 2] la FIGURE 2 une représentation schématique, en vue de dessous, de la face du capteur de proximité comprenant les électrodes de mesure illustrant différents agencements des couples d'électrodes de mesure d'un capteur de proximité selon l'invention,
[Fig. 3A] la FIGURE 3A illustre plusieurs images issues d'une simulation numérique du déplacement latéral de la tête fonctionnelle au-dessus d'un patient à imager ; le dispositif de détection est monté sur une tête fonctionnelle et les déplacements de la tête fonctionnelle sont contrôlés à partir des signaux de détection du dispositif de détection d'objets,
[Fig. 3B] la FIGURE 3B illustre plusieurs images, dans la continuité des images illustrées sur la FIGURE 3A, issues de la simulation numérique du déplacement latéral de la tête fonctionnelle au-dessus d'un patient à imager ; le dispositif de détection est monté sur une tête fonctionnelle et les déplacements de la tête fonctionnelle sont contrôlés à partir des signaux de détection du dispositif de détection d'objets,
[Fig. 4] la FIGURE 4 est une représentation schématique, en vue de dessous, de la face du capteur de proximité comprenant les électrodes de mesure illustrant un perfectionnement du capteur de proximité selon l'invention,
[Fig. 5] la FIGURE 5 est une représentation schématique, en vue de dessous, de la face du capteur de proximité comprenant les électrodes de mesure illustrant un perfectionnement du capteur de proximité selon l'invention,
[Fig. 6] la FIGURE 6 est une représentation schématique, en vue de dessous, de la face du capteur de proximité comprenant les électrodes de mesure illustrant un perfectionnement du capteur de proximité selon l'invention,
[Fig. 7] la FIGURE 7 est une représentation schématique en vue de coupe du capteur de proximité qui est monté sur une tête fonctionnelle.

### Description des modes de réalisation

Les modes de réalisation décrits ci-après étant nullement limitatifs, on pourra notamment considérer des variantes de l'invention ne comprenant qu'une sélection de caractéristiques décrites, isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

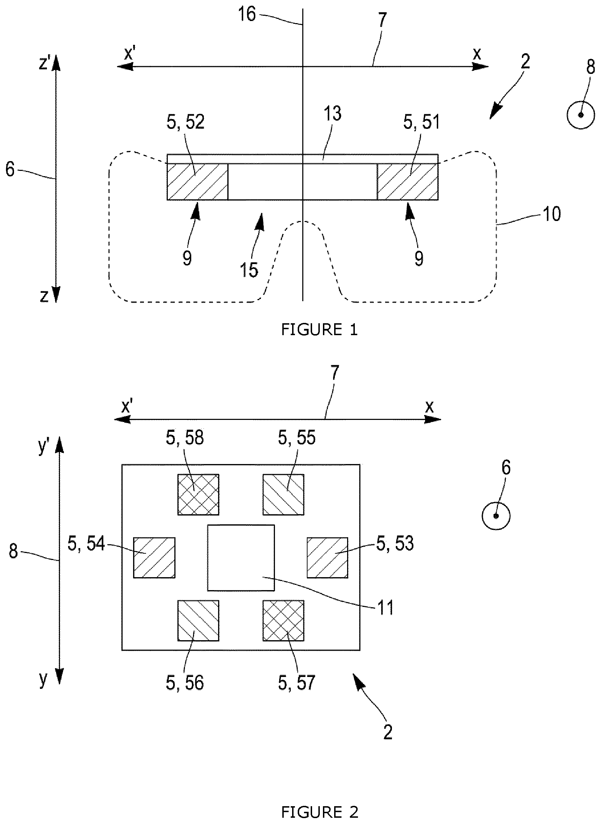

En référence à la FIGURE 1, il est présenté un mode de réalisation du dispositif de détection d'objets 1 selon l'invention. Le dispositif de détection d'objets 1 comprend un capteur de proximité 2. Le capteur de proximité 2 comprend au moins deux électrodes de mesure 5. Chaque électrode de mesure 5 du capteur de proximité est comprise sur une même face 15 du capteur de proximité 2. Aucune électrode de mesure 5 n'est située sur une face latérale du capteur de proximité 2. Dans la présente description, le terme « électrode » utilisé seul désigne une électrode de mesure 5. Deux électrodes de mesure 5 sont décalées le long de la direction latérale 7. Il est entendu par décalées, le fait qu'une des électrodes 5 soit situé en amont par rapport à l'autre pour un sens de déplacement donné. Selon le sens de déplacement x, l'électrode 51 est située en amont de l'électrode 52. A l'inverse pour le sens de déplacement x', l'électrode 52 est située en amont de l'électrode 51. Bien que cela constitue un mode de réalisation privilégié, il n'est pas nécessaire que les électrodes 5 soient alignées selon la direction latérale 7 pour que la détection d'un objet selon cette direction latérale 7 soit possible. Le dispositif de détection d'objets 1 comprend, en outre, une unité de traitement agencée pour détecter, par analyse des signaux provenant des deux électrodes de mesure 51, 52, la présence d'un objet situé verticalement 6 et/ou latéralement par rapport au détecteur de proximité 2.

Le signal mesuré par une électrode de mesure 5 est représentatif de la capacité électrode-objet mesurée par l'électrode de mesure 5. Le décalage selon la direction latérale 7 des deux électrodes 51, 52 du capteur de proximité 2 permet que la capacité électrode-objet mesurée par chaque électrode 51, 52 soit différente pour tout déplacement latéral d'un objet et/ou du capteur de proximité 2 l'un relativement à l'autre. Le décalage selon la direction latérale 7 des deux électrodes 51, 52 du capteur de proximité 2 permet que la capacité électrode-objet mesurée par chaque électrode 51, 52 soit différente pour tout déplacement vertical d'un objet et/ou du capteur de proximité 2 l'un relativement à l'autre.

En référence à la FIGURE 2, il est illustré plusieurs exemples dans lesquels les deux électrodes 5 de chaque couple d'électrodes 53-54, 55-56 et 57-58 sont décalées l'une par rapport à l'autre selon la direction latérale 7. Dans ces exemples non limitatifs, les couples d'électrodes 55-56 et 57-58 58 sont décalées l'une par rapport à l'autre selon la direction latérale 8 alors que, bien que cela soit envisageable, les électrodes du couple 53-54 ne sont pas décalées l'une par rapport à l'autre selon la direction latérale 8.

Aussi, lorsque le capteur de proximité 2, et donc les électrodes de mesure 5, est immobile, il est possible, à partir de la capacité électrode-objet mesurée par chacune des deux électrodes de mesure 51, 52, de détecter un objet présent dans la zone de détection 10 du capteur de proximité 2 et de déterminer une zone d'espace approximative dans laquelle se situe l'objet et/ou un mouvement approximatif de l'objet sans aucune électrode 5 disposée sur une face latérale du capteur de proximité 2. Cette détection d'objet et/ou cette détermination approximative de la position de l'objet et/ou du mouvement de l'objet repose sur l'analyse des signaux de mesure des deux électrodes de mesure 51, 52. L'étape d'analyse est détaillée plus bas.

Toujours dans le cas où le capteur de proximité 2 est immobile, afin d'affiner et d'obtenir une meilleure précision quant à la position approximative de l'objet et/ou quant à la détermination approximative du mouvement de l'objet, il est avantageux que le capteur de proximité 2 comprenne plus de deux électrodes 5. En particulier, il est préférable que le capteur de proximité 2 comprenne trois électrodes 5 ou, de préférence encore, quatre électrodes 5. Cela offre un bon compromis entre précision, coût du capteur de proximité 2 et coût de l'unité de traitement. Un nombre d'électrodes de mesure 5 trop important induit une augmentation directe du coût du capteur de proximité 2, une plus grande complexité dans le procédé de traitement des signaux de mesure et requiert une unité de traitement plus performante pour traiter les données dans un laps de temps compatible avec les exigences. Selon un perfectionnement avantageux de l'invention, le capteur de proximité 2 comprend trois électrodes de mesure 5 disposées de sorte à former un triangle, de préférence un triangle rectangle.

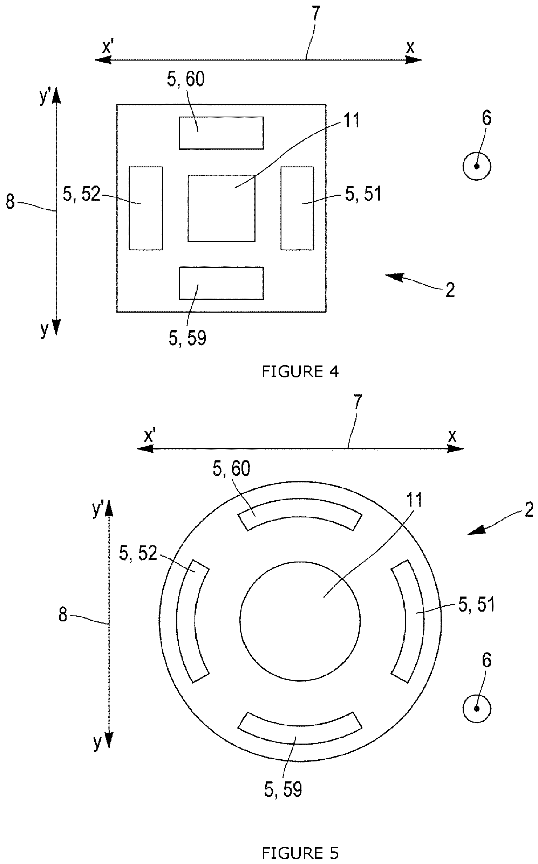

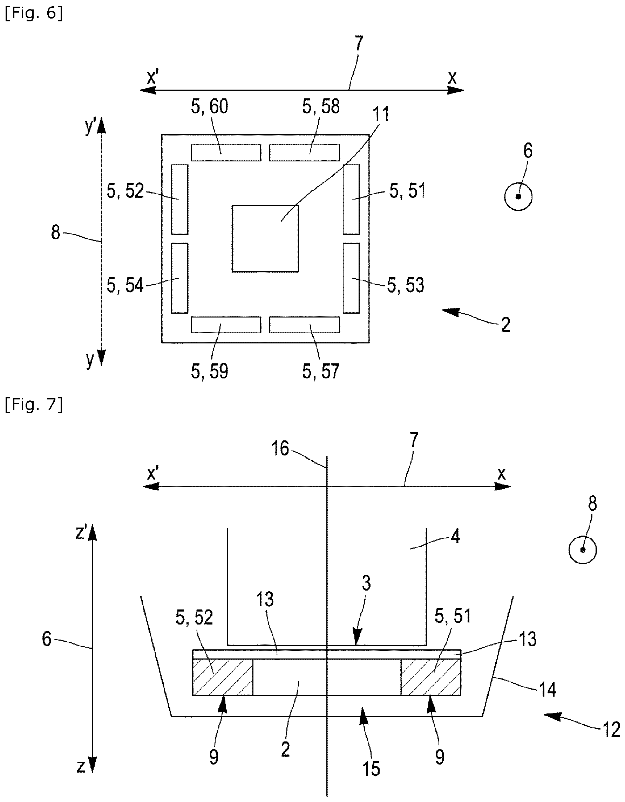

Dans des modes de réalisation avantageux présentés sur les FIGURES 4 à 6, le capteur de proximité 2 comprend plus de deux électrodes 5 pour optimiser la résolution spatiale par rapport au plus petit objet (ou relief) à détecter. Par exemple, il est possible de mesurer l'approche et la position d'une main, d'un bras, d'une petite tête d'enfant. En référence aux FIGURES 4 et 5, le capteur de proximité 2 comprend quatre électrodes de mesure 5 parmi lesquelles deux électrodes 51, 52 sont décalées et alignées selon la direction latérale 7, dite première direction latérale 7, et deux électrodes 59, 60 sont décalées et alignées selon la direction latérale 8, dite deuxième direction latérale 8. Dans un perfectionnement avantageux de l'invention présenté sur la FIGURE 6, le capteur de proximité 2 comprend huit électrodes de mesure 5 parmi lesquelles deux couples d'électrodes 51-52 et 53-54 sont décalées et alignées selon la première direction latérale 7 et deux couples d'électrodes 57-58 et 59-60 sont décalées et alignées selon la deuxième direction latérale 8.

Les signaux provenant des électrodes capacitives 5 sont interrogées individuellement et/ou de manière groupées en sommant ou combinant les signaux ou de manière différenciées en réalisant la différence entre signaux individuels, entre signaux individuels et signaux groupés ou entre signaux groupés. Cette approche est rendue possible en interrogeant une électrode 5 sans interférence avec les électrodes 5 de son voisinage. Par exemple, lorsqu'un objet est détecté, par exemple, par l'électrode 5, 59, du côté du capteur de proximité 2 en vis-à-vis du sens y de la direction 8, l'unité de traitement va, entre autres et à titre d'exemple non limitatif, comparer le signal de l'électrode individuelle 5, 60 avec les signaux groupés et/ou différenciés du groupe d'électrodes 5, 52, 51, 59 et comparer le signal de l'électrode individuelle 5, 59 avec les signaux groupés et/ou différenciés du groupe d'électrodes 5, 51, 52, 60. La résolution spatiale et la discrimination sont ainsi augmentées.

Comme cela est détaillé plus bas, dans le cas où le capteur de proximité 2 est déplaçable ou mobile, même lors d'un déplacement minime, il est alors possible de déterminer avec précision la position et/ou le mouvement d'un objet présent dans la zone détection 10 du capteur de proximité 2. Dans ce cas, l'unité de traitement est agencée pour déterminer, par analyse des signaux provenant des électrodes de mesure 5 du capteur de proximité 2 et de données relatives à un déplacement du capteur de proximité 2, la position relative et/ou le mouvement relatif d'un objet.

Sur les FIGURES 2 et 4 à 6, il est présenté la face 15 du capteur de proximité 2 comprenant les électrodes de mesure 5. La face de détection 9 est illustrée et comprise dans le plan 7, 8 des FIGURES 2 et 4 à 6. La face de détection 9 des électrodes capacitives 5 est destinée à être positionnée en direction de l'objet à imager, c'est-à-dire selon le sens z de la direction 6, de sorte à former la zone de détection 10 représentée sur la FIGURE 1. Cette direction 6 est dite verticale sans qu'elle ne limite le positionnement de la face de détection 9 orthogonalement à cette direction. Dans certains cas, la position de l'objet à imager, par exemple dans le cas d'un patient en position debout ou verticale, pourrait nécessiter que la face de détection 9 des électrodes capacitives, et donc la face 15 du capteur de proximité 2 comprenant les électrodes de mesure 5, soit positionnée orthogonalement à la direction horizontale. Le capteur de proximité 2 ne comprend pas d'électrode capacitive 5 destinée à être disposée sur les faces latérales de la tête fonctionnelle 4. Autrement dit, aucune des électrodes capacitives 5 du capteur de proximité 2 n'est disposée de sorte que sa face de détection 9 soit destinée à être principalement positionnée en regard du sens x, x', y ou y'.

Dans une amélioration du dispositif de détection d'objets 1, les deux électrodes de mesure 5 de chaque couple d'électrodes de mesure 51-52 et 59-60 sont alignées, respectivement, selon la première direction latérale 7 et selon la deuxième direction latérale 8.

Le capteur de proximité 2 comprend une ouverture centrale 11 formant un passage pour le faisceau émis par la tête fonctionnelle 4. Dans une amélioration du dispositif de détection d'objets 1, les deux électrodes de mesure 5 de chaque couple d'électrodes de mesure 51-52 et 59-60 sont disposées de part et d'autre de l'ouverture centrale 11. Espacer les deux électrodes capacitives 5 d'un couple d'électrodes de mesure 51-52 et 59-60 le long de l'axe reliant ces électrodes 5, et selon lequel elles sont alignées, permet d'améliorer la sensibilité du détecteur de proximité 2 en améliorant la discrimination et la résolution spatiale par augmentation de la différence des amplitudes des signaux des électrodes 5 et en augmentant la zone de détection de chaque électrodes 5 et de la zone de détection 5 du capteur de proximité 2.

Dans une amélioration du dispositif de détection d'objets 1, les électrodes de mesure 5 sont disposées en périphérie du capteur de proximité 2 de sorte à améliorer la sensibilité de la détection latérale 7, 8 du dispositif de détection 1.

Selon un mode de réalisation non limitatif, le dispositif de détection d'objets 1 est destiné à être monté sur une tête fonctionnelle 4. De manière générale, le dispositif de détection d'objets 1 est destiné à être monté sur tout moyen, dispositif ou système, pouvant être immobile ou mobile, nécessitant une détection d'objets environnants pour réaliser, de préférence de manière dynamique, un contournement de l'objet, un positionnement relativement à l'objet, un maintien à une distance donnée de l'objet, un évitement de l'objet ou empêcher ou prévenir une collision avec l'objet.

A cet effet, dans une amélioration du dispositif de détection d'objets 1, les électrodes de mesure 5 peuvent s'étendre au-delà de la face d'émission 3 de la tête fonctionnelle 4 de sorte à améliorer la sensibilité de détection latérale 7, 8 du dispositif de détection 1.

Dans une autre amélioration du dispositif de détection d'objets 1, le dispositif de détection d'objets comprend une ou plusieurs électrodes de garde 13 pour garder électriquement la ou les électrodes de mesure 5. L'électrode de garde 13 est polarisée. Cette ou ces électrodes de gardes 13 permettent ainsi de garder électriquement les électrodes de mesure 5 en évitant la formation de capacités parasites ou de couplage indésirables avec l'environnement. Le ou les électrodes de garde 13 sont disposées sur la face du capteur de proximité 2 qui est en regard de la face d'émission 3 de la tête ou fonctionnelle 4 ou qui est opposée à la face de détection 9 des électrodes de mesure 5. Les électrodes de mesure 5 sont gardées à l'arrière afin de supprimer ou réduire l'effet capacitif de la tête fonctionnelle et/ou de système d'imagerie 12 sur lequel est destiné à être montée le dispositif de détection 1. Une telle configuration permet d'optimiser la sensibilité des électrodes de mesure 5 en direction de l'objet à imager en délimitant la zone de détection 10 des électrodes de mesure 5 en direction de l'objet à imager.

Le dispositif de détection d'objet peut également comprendre un capot 14 en matériau diélectrique.

Il est également décrit le procédé de détection d'objets qui est mis en oeuvre au moyen du dispositif de détection d'objets 1 selon le premier mode de réalisation. Le procédé comprend l'étape consistant à mesurer le signal de mesure provenant de l'électrode 51 et le signal de mesure provenant de l'électrode 52. L'étape de mesure du signal est connue de l'homme du métier et elle est, par exemple, décrite dans le document WO2004/023067. Le procédé comprend également l'étape consistant à analyser, au moyen de l'unité de traitement du dispositif de détection 1, les deux signaux distincts provenant des deux électrodes de mesure 51, 52 pour détecter la présence d'un objet situé verticalement et/ou latéralement par rapport au capteur de proximité 2. Même dans le cas où le capteur de proximité 2 est immobile, le procédé permet une détermination approximative de la position de l'objet et/ou une détermination approximative du mouvement de l'objet. La détection d'objet et/ou la détermination approximative de la position de l'objet et/ou du mouvement de l'objet est réalisée en analysant et en comparant la non-différence et/ou la différence entre les signaux provenant des électrodes de mesure 5. De manière encore avantageuses, il est possible de sommer tout ou partie des signaux individuels pour un obtenir un ou des signaux, dit groupés, et de soustraire tout ou partie des signaux individuels pour obtenir un ou des signaux, dit différenciés. Ensuite, l'analyse et la comparaison croisée entre les signaux individuels, les signaux groupés et les signaux différenciés permet d'améliorer la détermination de la position approximative de l'objet et/ou le mouvement de l'objet lorsque le capteur de proximité est immobile.

Les dispositifs de détection d'objets à base d'électrodes capacitives de l'état de l'art utilisent, pour chaque sens de déplacement, au moins une électrode capacitive dont la zone de détection est orientée face à un sens de déplacement, en particulier vertical et latéral. Dans ce cas, pour un sens de déplacement donné, c'est le signal en provenance de l'électrode capacitive dont la zone de détection est orientée face au sens de déplacement donné qui est analysé. Ces dispositifs sont donc coûteux compte tenu du nombre d'électrodes nécessaires. En outre, la présence d'électrodes capacitives sur chaque face latérale des têtes fonctionnelles sur lesquelles elles sont montées induit un encombrement important. Selon l'invention, pour une direction de déplacement donnée, c'est l'analyse simultanée des signaux individuels, groupés et différenciés provenant des électrodes de mesure 5, associé à l'agencement particulier des électrodes de mesure 5 selon l'invention, qui permet de discriminer la présence et la position, verticale ou horizontale, d'un objet par rapport au détecteur de proximité 2.

En référence à la FIGURE 7, il est présenté un système 12 selon l'invention. Selon le mode de réalisation particulier, le système 12 est un système d'imagerie 12. Le système d'imagerie 12 comprend une tête fonctionnelle 4 sur laquelle est montée le dispositif de détection 1 selon l'invention. Selon un mode de réalisation préféré non limitatif, le système d'imagerie 12 est une machine médicale, en particulier pour de l'imagerie médicale. La tête fonctionnelle 4 comprend une face d'émission 3 qui est destinée à être positionnée en direction d'un objet à imager, par exemple un patient. Le capteur de proximité 2 est monté sur la face d'émission 3 de la tête fonctionnelle 4. Lorsque la face d'émission 3 est plane, et le patient en position allongée, la face d'émission 3 est destinée à être parallèle au plan horizontal, au plan formé par les axes 7, 8, et à être perpendiculaire à l'axe vertical 6. La tête fonctionnelle 4 est déplaçable selon la direction 6 qui est orthogonale à la face 15 du capteur de proximité 2 comprenant les électrodes de mesure 5 et qui est orthogonale à la face d'émission 3 de la tête fonctionnelle 4. La tête fonctionnelle 4 est également déplaçable latéralement 7, 8 selon toute direction latérale. Le capteur de proximité 2 se déplace donc avec la tête fonctionnelle 4. Outre les déplacements rectilignes ou par translation, la tête fonctionnelle 4 est aussi déplaçable par rotation. Un déplacement par rotation peut être décomposé ou équivalent à un déplacement selon deux directions de l'espace, par exemple, un axe vertical et un axe horizontal. Aussi, afin de faciliter la description du mode de réalisation, seul les déplacements verticaux et horizontaux sont décrits.

Le système 12 comprend unité de traitement qui est, de préférence, celle du dispositif de détection 1 ou, à défaut, qui est configurer de manière équivalente à celle du dispositif de détection d'objets 1. Aussi, l'unité de traitement est agencée pour détecter, par analyse des signaux provenant d'au moins deux électrodes de mesure 5 du capteur de proximité 2 selon l'invention, la présence d'un objet situé latéralement 7, 8 et verticalement 6 par rapport au détecteur de proximité 2 et/ou par rapport à la tête fonctionnelle 2, en particulier, mais pas uniquement, par rapport à la face d'émission 3 de la tête fonctionnelle 2.

L'unité de traitement est agencée, en outre, pour, à partir des signaux provenant des électrodes de mesure 5 du capteur de proximité 2 et de données relatives à un déplacement de la tête fonctionnelle 4, déterminer la position et/ou le mouvement d'un objet relativement au capteur de proximité 2 et à la tête fonctionnelle 2, en particulier, mais pas uniquement, par rapport à la face d'émission 3 de la tête fonctionnelle 2. Lorsque le capteur de proximité 2 est immobile, le mouvement relatif de l'objet par rapport à la tête fonctionnelle 2 est déterminé et la position de l'objet relativement au capteur de proximité 2 est généralement déterminée approximativement dans une zone d'espace. Aussi, la prise en compte de données relatives au déplacement de la tête fonctionnelle 2, et donc du capteur de proximité 2, permet d'améliorer la détermination de la position et du mouvement relatif de l'objet par rapport à la tête fonctionnelle 2 sans qu'il soit nécessaire de disposer d'électrodes 5 sur une face latérale ou une extrémité latérale ou un côté latéral ou un bord latéral du capteur de proximité 2.

Selon une amélioration de l'invention, le système d'imagerie 12 comprend une unité de commande. L'unité de commande du système d'imagerie 12 est agencée pour commander et/ou contrôler des moyens de mise en mouvement de la tête fonctionnelle 4 à partir des signaux provenant du capteur de proximité 2 et/ou de données provenant de l'unité de traitement du dispositif de détection 1. Dans ce cas, l'unité de commande du système d'imagerie 12 est agencée pour, à partir des signaux provenant des électrodes de mesure 5 du capteur de proximité 2 et/ou des données provenant de l'unité de traitement du dispositif de détection 1, empêcher la collision de la tête fonctionnelle 4 avec un objet environnant. Dans ce cas, l'unité de commande du système d'imagerie 12 est agencée, en outre, pour, à partir des signaux provenant des électrodes de mesure 5 du capteur de proximité 2 et/ou des données provenant de l'unité de traitement du dispositif de détection 1, positionner le capteur de proximité 2 à une distance donnée de l'objet à imager et/ou maintenir une distance minimale entre la tête fonctionnelle 2, en particulier durant le déplacement de la tête fonctionnelle 4 relativement à l'objet, et l'objet à imager et/ou immobiliser la tête fonctionnelle 2 en cas de risque de collision.

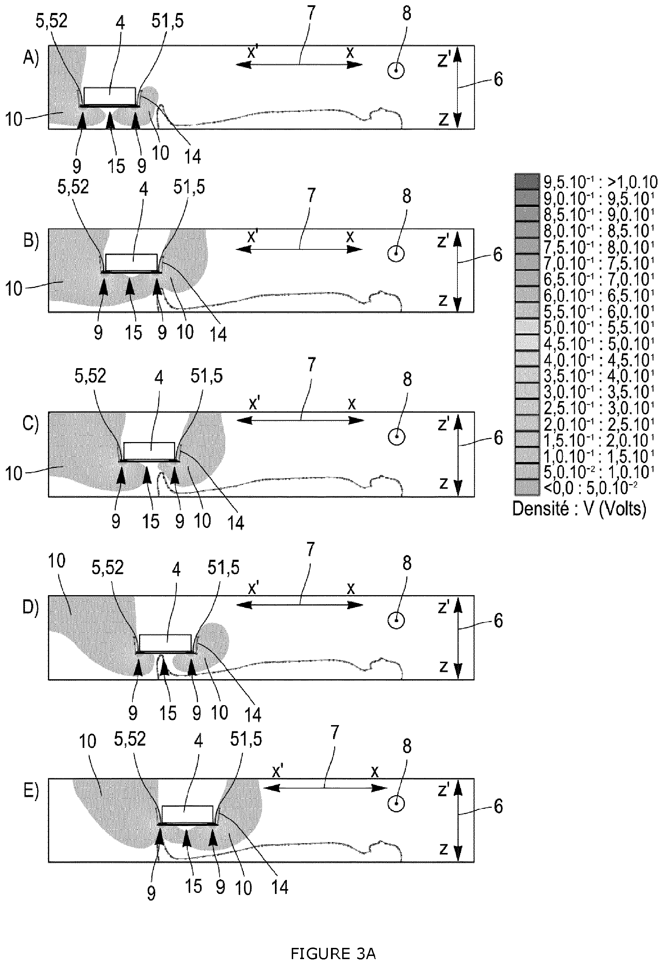

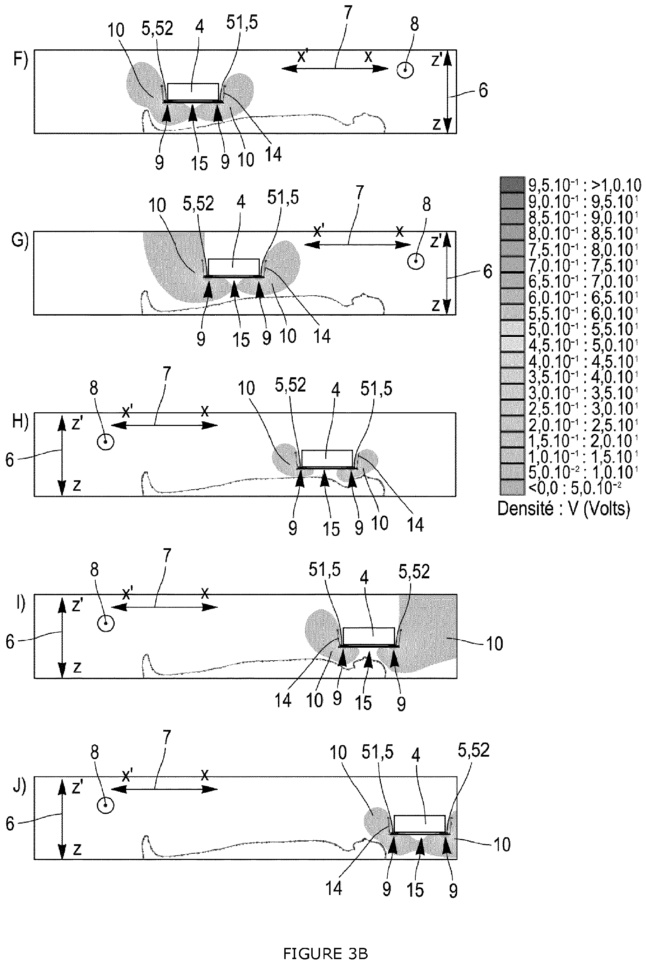

Sur les FIGURES 3A et 3B sont illustrées différentes positions du système 12 comprenant une tête fonctionnelle 4 sur laquelle est montée le dispositif de détection 1 selon le premier mode de réalisation. Les schémas des FIGURES 3A et 3B proviennent d'une simulation effectuée en générant le mouvement vertical de la tête fonctionnelle 4 équipée du dispositif de détection d'objets 1. Dans le mode de réalisation particulier, lorsque la taille de l'objet à imager, ici un patient, est supérieure à la taille de la tête fonctionnelle 2, et en particulier à l'espacement entre les électrode 5 de mesure, les valeurs de capacité électrode-objet mesurées par les électrodes 5 varient peu d'une électrode 5 à l'autre lors d'un déplacement latéral (7,8). Un des objectifs est, ici, de détecter le mouvement d'un objet pour empêcher une éventuelle collision avec la tête fonctionnelle. Un déplacement selon un axe latéral 7, 8, tel qu'effectué lors du scan du patient, est imprimé à la tête fonctionnelle 4 et un mouvement asservi verticalement 6 afin de conserver la distance minimale requise. Cette distance est obtenu en pilotant une simulation électrostatique par éléments finis, simplifiée en 2D, dont les paramètres de définition de la géométrie sont la position des détecteurs 51, 52 et donc les paramètres de sortie que sont les valeurs des capacités électrodes-objet entre la cible, ici un corps humain, et les électrodes capacitives 51, 52. Les valeurs de capacité électrode-objet sont ensuite transformées en valeurs de distance via une calibration initiale (elle aussi réalisée par éléments finis), ces valeurs de distance étant réinjectées dans le calcul de l'asservissement vertical de la tête fonctionnelle 4. Dans cet exemple, la tête fonctionnelle 4 se déplace dans la direction 7 selon le sens x. La zone de détection 10, ainsi que les lignes de champs, de chacune des deux électrodes 51, 52 est également représentée. Sur l'image A de la FIGURE 3A, la tête fonctionnelle 4 est en déplacement selon le sens x et le détecteur 51 voit la capacité électrode-objet mesurée augmentée sans que le détecteur 52 ne détecte la présence d'un objet. Bien qu'à ce moment précis la détermination de la position de l'objet par rapport au dispositif de détection 1 ne soit pas possible, la variation de la capacité électrode-objet mesurée par le détecteur 51 lors de la poursuite du déplacement selon x associé à la détection, et le cas échéant à la variation de la capacité électrode-objet mesurée par le détecteur 52, ou à la non détection d'objet par le détecteur 52 va permettre de détecter, a minima, mais aussi de déduire la position de l'objet et de déterminer le mouvement relatif de l'objet, en particulier, mais pas uniquement, selon la direction latérale 7. Toujours en référence à l'image A de la FIGURE 3A, lorsque la capacité électrode-objet mesurée par le détecteur 51 augmente, le déplacement de la tête fonctionnelle 4 selon l'axe 6 dans le sens z' induit une variation de la capacité mesurée qui permettra de déterminer la position de l'objet. Si la capacité mesurée par le détecteur 51 reste constante lors du déplacement selon l'axe 6 dans le sens z' cela implique que l'objet est bien situé latéralement dans le sens x par rapport au dispositif de détection 1 alors que si elle diminue cela indique que l'objet à au moins une partie qui est située verticalement selon l'axe 6 dans le sens z'. Le même fonctionnement est transposable au cas où les deux capteurs 51, 52 détecte la présence d'un objet. Cet exemple, volontairement simple, d'analyse des signaux provenant des deux électrodes de mesure 5 est transposable et généralisable au cas où le capteur de proximité 2 comprend trois électrodes ou plus. Dans ce cas, l'analyse et la comparaison croisée entre les signaux individuels, les signaux groupés et les signaux différenciés des électrodes de mesure 5 permettra de déterminer plus rapidement et plus précisément la position d'un objet même dans le cas où le capteur de proximité 2 est immobile.

De manière préférée, il est également possible de déterminer qu'un objet est situé latéralement à la tête fonctionnelle 4, et également de déterminer la position latérale d'un objet situé latéralement à la tête fonctionnelle 4, en déterminant, entre autres et à titre d'exemple non limitatif, le différentiel observé entre la variation du signal électrode-objet mesuré par l'électrode 51 et la variation du signal électrode-objet observé par l'électrode 52. De manière également préférée, il est également possible de déterminer qu'un objet est situé verticalement à la tête fonctionnelle 4, et également de déterminer la position verticale d'un objet situé latéralement à la tête fonctionnelle 4, et/ou le mouvement de l'objet s'il se déplace, en observant qu'il n'y a pas variation du signal capacitif mesuré par l'électrode 51 et qu'il n'y a pas variation du signal mesuré par l'électrode 52. Comme décrit plus haut, l'analyse et la comparaison croisée entre les signaux individuels, les signaux groupés et les signaux différenciés des électrodes de mesure 5 permet d'obtenir la position de l'objet par rapport au capteur de proximité 2 ainsi que le mouvement relatif de l'objet.

Bien sûr, l'invention n'est pas limitée aux exemples qui viennent d'être décrits et de nombreux aménagements peuvent être apportés à ces exemples sans sortir du cadre de l'invention.

De plus, les différentes caractéristiques, formes, variantes et modes de réalisation de l'invention peuvent être associés les uns avec les autres selon diverses combinaisons dans la mesure où ils ne sont pas incompatibles ou exclusifs les uns des autres.

## Revendications

1. Dispositif de détection d'objets (1) comprenant un capteur de proximité (2) , ledit capteur de proximité comprend deux électrodes capacitives de mesure (5), chacune des électrodes de mesure du capteur de proximité sont comprises sur une même face (15) du capteur de proximité, les deux électrodes de mesure sont décalées le long d'une direction latérale (7, 8),
ledit dispositif de détection d'objets comprend, en outre, une unité de traitement agencée pour détecter, par analyse des signaux provenant des deux électrodes de mesures décalées le long de la direction latérale, la présence d'un objet situé latéralement par rapport au capteur de proximité.

2. Dispositif (1) selon la revendication 1, dans lequel :
- le capteur de proximité (2) comprend au moins trois électrodes de mesure (5) parmi lesquelles trois électrodes de mesure sont disposées de sorte à former un triangle,
- l'unité de traitement est agencée pour détecter, par analyse des signaux provenant d'au moins deux électrodes de mesure parmi les trois électrodes formant un triangle, la présence d'un objet situé latéralement par rapport au détecteur de proximité.

3. Dispositif (1) selon la revendication 1 ou 2, dans lequel l'unité de traitement est agencée pour détecter, par analyse des signaux provenant d'au moins deux électrodes de mesure (5) du capteur de proximité (2), une direction de déplacement relatif d'un objet.

4. Dispositif (1) selon l'une quelconque des revendications précédentes, dans lequel le capteur (2) de proximité est déplaçable selon une direction (6), dite verticale, orthogonale à la face (15) du capteur de proximité comprenant les électrodes de mesure (5) et est déplaçable latéralement (7,8).

5. Dispositif (1) selon l'une quelconque des revendications 1 à 3, dans lequel le capteur (2) de proximité est destiné à :
- être monté sur une tête fonctionnelle (4) déplaçable selon une direction (6), dite verticale, orthogonale à la face (15) du capteur de proximité comprenant les électrodes de mesure (5) et déplaçable latéralement (7, 8), et
- être disposé en regard d'une face (3), dite face d'émission (3), de la tête fonctionnelle ; ladite face d'émission de la tête fonctionnelle est destinée à être positionnée en direction d'un objet à imager ou à analyser.

6. Dispositif (1) selon l'une quelconque des revendications précédentes, dans lequel le capteur de proximité (2) comprend une ouverture centrale 11 ; au moins deux électrodes de mesure (5) sont disposées de part et d'autre de l'ouverture centrale.

7. Dispositif (1) selon la revendication précédente, dans lequel le capteur de proximité (2) est agencé pour qu'une, plusieurs ou l'ensemble des électrodes de mesure (5) s'étendent radialement, par rapport à l'ouverture centrale (11), au-delà de la face d'émission (3) de la tête fonctionnelle (4).

8. Dispositif (1) selon l'une quelconque des revendications précédentes, dans lequel une, plusieurs ou l'ensemble des électrodes de mesure (5) sont disposées en périphérie du capteur de proximité (2).

9. Dispositif (1) selon l'une quelconque des revendications précédentes, dans lequel l'unité de traitement est agencée pour détecter la présence d'un objet par analyse :
- de signaux individuels provenant d'au moins deux électrodes de mesure (5) ou de chacune des électrodes de mesure du capteur de proximité (2), et/ou
- de signaux groupés obtenus en sommant des signaux provenant d'au moins deux électrodes de mesure (5) ou de chacune des électrodes de mesure du capteur de proximité, et/ou
- de signaux différenciés obtenus en soustrayant des signaux provenant d'au moins deux électrodes de mesure (5) ou de chacune des électrodes de mesure du capteur de proximité, et/ou
- de signaux individuels et/ou de signaux groupés et/ou de signaux différenciés.

10. Dispositif (1) selon l'une quelconque des revendications 4 à 9, dans lequel l'unité de traitement est agencée pour, à partir des signaux provenant des électrodes de mesure (5) du capteur de proximité (2) et de données relatives à un déplacement du capteur de proximité, déterminer la position d'un objet et/ou le déplacement d'un objet relativement au capteur de proximité.

11. Système (12) comprenant :
- une tête fonctionnelle (4) comprenant une face d'émission (3) destinée à être positionnée en direction d'un objet à imager ou à analyser,
- un capteur de proximité (2) monté sur la face d'émission de la tête fonctionnelle, ledit capteur de proximité comprend au moins deux électrodes capacitives de mesure (5) parmi lesquelles deux électrodes de mesure sont décalées le long d'une direction latérale (7,8), chacune des électrodes de mesure du capteur de proximité sont comprises sur une même face (15) du capteur de proximité,
la tête fonctionnelle est déplaçable selon une direction (6), dite verticale,
orthogonale à la face du capteur comprenant les électrodes de mesure (5) et déplaçable latéralement (7,8),
ledit système comprend une unité de traitement agencée pour détecter, par analyse des signaux provenant des deux électrodes de mesure décalées le long de la direction latérale, la présence d'un objet situé latéralement par rapport au détecteur de proximité.

12. Système (12) selon la revendication précédente, dans lequel l'unité de traitement est agencée pour détecter, par analyse des signaux provenant des au moins deux électrodes de mesures (5), une direction de déplacement relatif d'un objet.

13. Système (12) selon la revendication 11 ou 12, dans lequel l'unité de traitement est agencée pour, à partir des signaux provenant des électrodes de mesure (5) du capteur de proximité (2) et de données relatives à un déplacement de la tête fonctionnelle, déterminer la position d'un objet et/ou le déplacement d'un objet relativement au capteur de proximité.

14. Système (12) selon l'une quelconque des revendications 11 à 13, comprenant une unité de commande agencée pour, à partir des signaux provenant des électrodes de mesure (5) du capteur de proximité (2), positionner le capteur de proximité à une distance donnée d'un objet à imager et/ou maintenir une distance minimale entre la tête fonctionnelle (4) et l'objet à imager et/ou immobiliser la tête fonctionnelle.

15. Procédé de détection d'objets mise en oeuvre par un dispositif de détection d'objets (1) comprenant un capteur de proximité (2), ledit capteur de proximité comprend deux électrodes capacitives de mesure (5), chacune des électrodes de mesure du capteur de proximité sont comprises sur une même face (15) du capteur de proximité, les deux électrodes de mesure sont décalées le long d'une direction latérale (7, 8) ; ledit procédé comprend les étapes consistant à :
- mesurer les signaux provenant des deux électrodes de mesure du capteur de proximité,
- analyser, par une unité de traitement du dispositif de détection, les deux signaux provenant des deux électrodes de mesure décalées le long de la direction latérale pour détecter la présence d'un objet situé latéralement par rapport au détecteur de proximité.

16. Procédé selon la revendication précédente, ledit procédé comprend les étapes consistant à :
- mesurer au moins deux signaux provenant chacun d'une électrode de mesure (5) distincte parmi au moins trois électrodes de mesure du capteur de proximité (2), trois électrodes de mesure du capteur de proximité sont disposées de sorte à former un triangle,
- analyser, par l'unité de traitement du dispositif de détection (1), les signaux provenant d'au moins deux électrodes de mesure parmi les trois électrodes du capteur de proximité formant un triangle pour détecter la présence d'un objet situé latéralement par rapport au détecteur de proximité.

17. Procédé selon la revendication 15 ou 16, comprenant l'étape de détermination d'une direction de déplacement relatif d'un objet par analyse, par l'unité de traitement du dispositif de détection (1), d'au moins deux signaux provenant de deux électrodes de mesure (5) distinctes du capteur de proximité (2).

18. Procédé de détection d'objets selon l'une quelconque des revendications 15 à 17, dans lequel la détection de la présence d'un objet situé latéralement par rapport au détecteur de proximité (2) est réalisée par analyse :
- de signaux individuels d'au moins deux électrodes de mesure (5) ou de chacune des électrodes de mesure du capteur de proximité, et/ou
- de signaux groupés obtenus en sommant des signaux provenant d'au moins deux électrodes de mesure ou de chacune des électrodes de mesure du capteur de proximité, et/ou
- de signaux différenciés obtenus en soustrayant des signaux provenant d'au moins deux électrodes de mesure ou de chacune des électrodes de mesure du capteur de proximité, et/ou
- de signaux individuels et/ou de signaux groupés et/ou de signaux différenciés.

19. Procédé de détection d'objets selon l'une quelconque des revendications 15 à 18, dans lequel le capteur de proximité (2) est déplaçable selon une direction (6), dite verticale, orthogonale à la face (15) du capteur comprenant les électrodes de mesure (5) et est déplaçable latéralement (7,8),
ledit procédé comprend l'étape consistant à déterminer la position d'un objet et/ou le déplacement d'un objet relativement au capteur de proximité par analyse des signaux provenant des électrodes de mesure du capteur de proximité et de données relatives à un déplacement du capteur de proximité.

20. Procédé de détection d'objets selon l'une quelconque des revendications 15 à 19, dans lequel le capteur de proximité (2) est destiné à être monté sur une tête fonctionnelle (4) et à être disposé en regard d'une face (3), dite face d'émission (3), de la tête fonctionnelle ; ladite tête fonctionnelle est destinée à être positionnée en direction d'un objet à imager ou à analyser et est déplaçable selon une direction (6), dite verticale, orthogonale à la face (15) du capteur comprenant les électrodes de mesure (5) et est déplaçable latéralement (7,8) ;
ledit procédé comprend l'étape consistant à, à partir des signaux provenant des électrodes de mesure du capteur de proximité, générer des données, par l'unité de traitement, destinées à être utilisées par une unité de commande, pour :
- maintenir une distance minimale entre le capteur de proximité et l'objet à imager, et/ou
- positionner le capteur de proximité à une distance donnée de l'objet à imager, et/ou
- immobiliser la tête fonctionnelle.
